# EUROPEAN PATENT SPECIFICATION

(11) **EP 4 484 946 B1**
(45) Date of publication and mention of the grant of the patent: **09.09.2026**
(21) Application number: 24185339.9
(22) Date of filing: 28.06.2024
(51) Int. Cl.: G01N 30/86

(54) **HEMOGLOBIN TYPE DETERMINATION METHOD, TRAINED MODEL GENERATION METHOD, TRAINED MODEL, HEMOGLOBIN ANALYSIS SYSTEM, AND PROGRAM**
HÄMOGLOBINTYPBESTIMMUNGSVERFAHREN, VERFAHREN ZUR ERZEUGUNG EINES TRAINIERTEN MODELLS, TRAINIERTES MODELL, HÄMOGLOBINANALYSESYSTEM UND PROGRAMM
PROCÉDÉ DE DÉTERMINATION DE TYPE D'HÉMOGLOBINE, PROCÉDÉ DE GÉNÉRATION DE MODÈLE ENTRAÎNÉ, MODÈLE ENTRAÎNÉ, SYSTÈME D'ANALYSE D'HÉMOGLOBINE ET PROGRAMME

(30) Priority: 29.06.2023 JP 2023107558
(43) Date of publication of application: 01.01.2025
(73) Proprietor: ARKRAY, Inc., Kyoto-shi, Kyoto 601-8045 (JP)
(72) Inventor: GOTO, Toshiki, Kyoto, 602-0008 (JP); KASAHARA, Junya, Kyoto, 602-0008 (JP); HIGASHIYAMA, Tetsuya, Kyoto, 602-0008 (JP); TORITSUKA, Kenji, Tokyo, 160-0004 (JP); KATO, Masaki, Kyoto, 602-0008 (JP)
(74) Representative: Dehns

(56) References cited:
- CN-A- 116 046 968
- DE-A1- 10 051 806

## Description

### BACKGROUND

### Technical Field

The present invention relates to a hemoglobin species determination method, a trained model generation method, a hemoglobin analysis system, and a program.

### Related Art

Conventionally, a method of measuring glycosylated hemoglobin (for example, HbA1a, HbA1b, and HbA1c) and mutant hemoglobin (also known as "variant hemoglobin", for example, HbF and HbS) of a measurement sample such as blood by using a liquid chromatography method has been proposed (refer to, for example, Japanese Patent No. 6856434, Japanese Patent No. 5948727, Japanese Patent No. 6492856, and Japanese Patent Application Laid-Open (JP-A) No. 2017-203677).

In the separation and fractionation methods disclosed in these patent documents, the separation time is lengthened in order to specify the species of hemoglobin in a blood sample, and determination is made on the basis of a peak pattern at a specific time, so that it takes time for measurement.

At least preferred embodiments of the invention provide a hemoglobin species determination method, a trained model generation method, a hemoglobin analysis system, and a program capable of specifying the species of hemoglobin in a blood sample with higher determination accuracy for the blood sample.

CN 116046968 A discloses a liquid chromatography workstation data processing method, comprising the steps of: obtaining the chromatographic data of the sample through the liquid chromatography workstation; preprocessing the chromatographic data; identifying the peak feature points and peak types of the preprocessed chromatographic data; inputting the preprocessed chromatographic data, peak feature points and peak types into the chromatographic data processing model, and outputting the content of each component of the sample.

DE 10051806 A1 discloses a method for identifying and characterizing microbial mixtures by means of infrared, Fourier transform infrared, Raman, Fourier transform Raman or gas chromatography analyses.

### SUMMARY

According to one aspect of the invention, there is provided a hemoglobin species determination method including preparing, using a computer, a trained model generated by using, as a learning data set for machine learning, a data set including learning frequency analysis data generated by performing frequency analysis on separation data generated by a separation process on a learning blood sample in which a species of hemoglobin is known and including the known species of hemoglobin; preparing, using a computer, test frequency analysis data by performing frequency analysis on separation data generated by a separation process on a test blood sample in which the species of hemoglobin is unknown; and determining, using a computer, the species of hemoglobin contained in the test blood sample by inputting the test frequency analysis data to the trained model. The separation data for the learning frequency analysis data and the separation data for the test frequency analysis data are chromatograms. The learning frequency analysis data and the test frequency analysis data are generated by performing frequency analysis on the separation data for the learning frequency analysis data and the separation data for the test frequency analysis data, the separation data for the learning frequency analysis data and the separation data for the test frequency analysis data having been subjected to a standardization process. The learning frequency analysis data and the test frequency analysis data are generated via wavelet analysis.

At least preferred embodiments provide a hemoglobin species determination method capable of specifying the species of hemoglobin in a blood sample even in a case in which separation data having a short separation time of a separation process on the blood sample is used.

At least preferred embodiments provide a hemoglobin species determination method capable of specifying the species of hemoglobin with higher determination accuracy for a blood sample.

### BRIEF DESCRIPTION OF THE DRAWINGS

Exemplary embodiments of the present invention will be described in detail based on the following figures, wherein:
Fig. 1 is a schematic configuration diagram of an HPLC apparatus;
Fig. 2 is a configuration diagram of a control system of the HPLC apparatus that is a trained model generation apparatus;
Fig. 3 is a flowchart for describing a trained model generation method according to an embodiment of the invention;
Fig. 4 is a diagram illustrating data used for machine learning for determining the species of hemoglobin, and is a diagram of a chromatogram obtained by measuring a blood sample with the HPLC apparatus;
Fig. 5 is a diagram illustrating data used for machine learning for determining the species of hemoglobin following Fig. 4, and is a diagram of a chromatogram as a result of performing a standardization process on the chromatogram in Fig. 4;
Fig. 6 is a diagram illustrating data used for machine learning for determining the species of hemoglobin following Fig. 5, and is a diagram obtained by performing wavelet transformation on a chromatogram as a result of performing the standardization process in Fig. 5;
Fig. 7 is a configuration diagram of a control system of an HPLC apparatus that is a hemoglobin determination apparatus;
Fig. 8 is a flowchart for describing a method of determining a species of hemoglobin contained in an unknown sample by using a trained model according to an embodiment of the invention;
Fig. 9A is a diagram for describing a shape of a peak in a case in which a blood sample is measured with the HPLC apparatus during different separation times, and is a chromatogram in a case in which hemoglobin E is measured;
Fig. 9B is a diagram for describing the shape of a peak in a case in which a blood sample is measured with an HPLC apparatus at different separation times, and is a chromatogram in a case in which abnormal hemoglobin A is measured;
Fig. 10A is a diagram for describing a shape of a peak in a case in which a blood sample is measured during a different separation time by an HPLC apparatus different from the HPLC apparatus used for measurement in Fig. 9, and is a chromatogram in a case in which hemoglobin E is measured;
Fig. 10B is a diagram for describing a shape of a peak in a case in which a blood sample is measured during a different separation time by an HPLC apparatus different from the HPLC apparatus used for the measurement in Fig. 9, and is a chromatogram in a case in which abnormal hemoglobin A is measured;
Fig. 11A is a diagram for describing a shape of a peak in a case in which the same blood sample is measured by different HPLC apparatuses, and is a chromatogram in a case in which the measurement time is long;
Fig. 11B is a diagram for describing a shape of a peak in a case in which the same blood sample is measured by different HPLC apparatuses, and is a chromatogram in a case in which a measurement time is short;
Fig. 12A is a diagram illustrating data used for machine learning for determining the species of hemoglobin according to another non-claimed embodiment of the invention, and is a diagram of an amplitude spectrum obtained by performing a Fourier transform on the chromatogram in Fig. 4; and
Fig. 12B is a diagram illustrating data used for machine learning for determining the species of hemoglobin according to another non-claimed embodiment of the invention, and is a diagram of a power spectrum obtained by performing a Fourier transform on the chromatogram in Fig. 4.

### DETAILED DESCRIPTION

Hereinafter, an embodiment of the invention will be described.

### (Configuration of Trained Model Generation Apparatus)

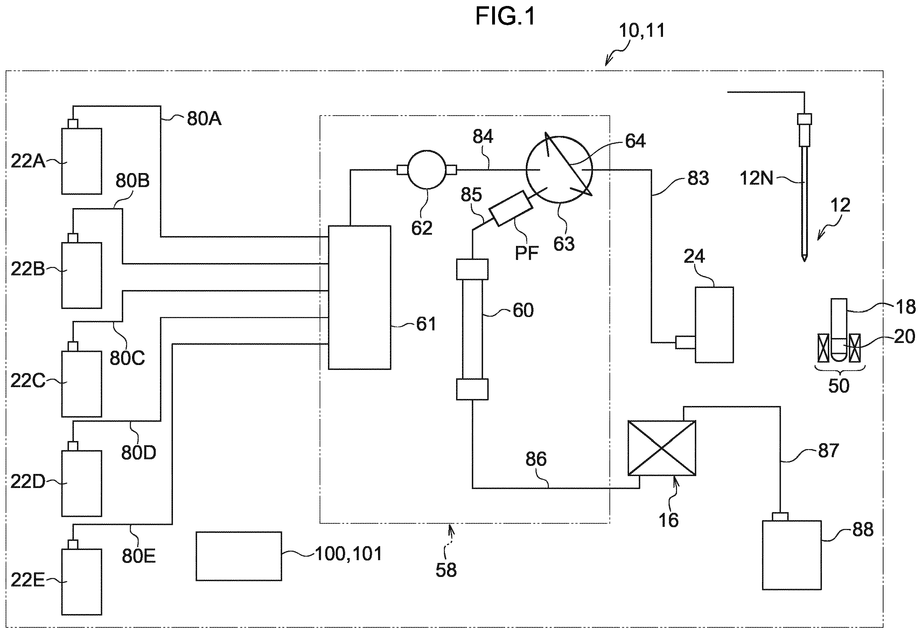

Fig. 1 is a schematic configuration diagram of a high performance liquid chromatography (HPLC) apparatus 10 using HPLC as an example of a trained model generation apparatus in the present embodiment. The trained model generation apparatus is an example of a hemoglobin analysis system.

The HPLC apparatus 10 is configured to automatically measure the concentrations of glycohemoglobin (for example, HbA1a, HbA1b, and HbA1c) and mutant Hb (for example, HbF and HbS) in whole blood from a set blood collection tube 18, and includes a sample preparation unit 12, an analysis unit 58, and a photometry unit 16.

The HPLC apparatus 10 includes a plurality of eluent bottles 22A, 22B, 22C, 22D, and 22E (five in Fig. 1). The eluent bottles 22A to 22E respectively holds eluents A to E to be supplied to an analytical column 60 that will be described later. For example, a composition, a component ratio, pH, an osmotic pressure, and the like of each eluent are different depending on the use.

The blood collection tube 18 is stored in, for example, a rack (not illustrated), and is configured to be moved to a position where blood can be collected by a nozzle 12N in the sample preparation unit 12 that will be described later.

The sample preparation unit 12 is used to prepare a sample to be introduced into the analytical column 60 from blood collected from the blood collection tube 18. The sample preparation unit 12 includes the nozzle 12N and a dilution tank 24.

The nozzle 12N is used to collect various liquids including a blood sample 20 of the blood collection tube 18, and can suck and discharge a liquid and is movable in a vertical direction and a horizontal direction. An operation of the nozzle 12N is controlled by the control unit 100 that will be described later.

The analysis unit 58 controls adsorption and desorption of components contained in a blood sample such as hemoglobin, which is a biological component, to and from a filler of the analytical column 60, and supplies various biological components to the photometry unit 16. A set temperature in the analysis unit 58 is, for example, about 40°C. The analytical column 60 holds a filler for selectively adsorbing a component contained in the sample. As the filler, for example, a methacrylic acid-methacrylic acid ester copolymer is used.

The analysis unit 58 includes a manifold 61, a liquid feeding pump 62, and an injection valve 63 in addition to the analytical column 60.

The manifold 61 is used to selectively supply an eluent from a specific eluent bottle among the plurality of eluent bottles 22A to 22E to the analytical column 60. The manifold 61 is connected to each of the eluent bottles 22A, 22B, 22C, 22D, and 22E via pipes 80A to 80E, and is connected to the injection valve 63 via a pipe 84.

The liquid feeding pump 62 is used to apply power for moving an eluent to the injection valve 63, and is provided in the middle of the pipe 84.

The injection valve 63 is capable of collecting a certain amount of introduction sample and introducing the introduction sample into the analytical column 60, and includes a plurality of introduction ports and discharge ports (not illustrated). An injection loop 64 is connected to the injection valve 63. The injection loop 64 can retain a certain amount (for example, several µL) of liquid, and by appropriately switching the injection valve 63, a state in which the injection loop 64 communicates with the dilution tank 24 and an introduction sample is supplied from the dilution tank 24 to the injection loop 64, and a state in which the injection loop 64 communicates with the analytical column 60 via a prefilter PF and the pipe 85 and an introduction sample is introduced from the injection loop 64 to the analytical column 60, can be selected. As such an injection valve 63, for example, a six-way valve may be used. The prefilter PF is a filter that filters a sample or an eluent.

The photometry unit 16 is used to optically detect hemoglobin contained in a sample supplied from the analytical column 60, and is connected to a waste liquid tank 88 that discharges the sample supplied from the analytical column 60 via a pipe 87.

Here, in a case in which the sample is introduced into the analytical column 60, a time during which the analytical column 60 retains a component contained in the sample varies for each component, and thus an amount of a component contained in the sample supplied to the photometry unit 16 varies with the elapse of time. In other words, components contained in the sample are separated by the analytical column 60.

Under the control of a control unit 100 that will be described later, the photometry unit 16 continuously measures the absorbance of a component contained in the sample supplied from the analytical column 60, and transmits a measurement result of the absorbance to the control unit 100.

Therefore, sample separation data is obtained by recording the measurement result of the absorbance transmitted from the photometry unit 16. That is, the analysis unit 58 is a member that performs a separation process in the present embodiment, and the analysis unit 58 and the photometry unit 16 are an example of a separation unit in the present embodiment.

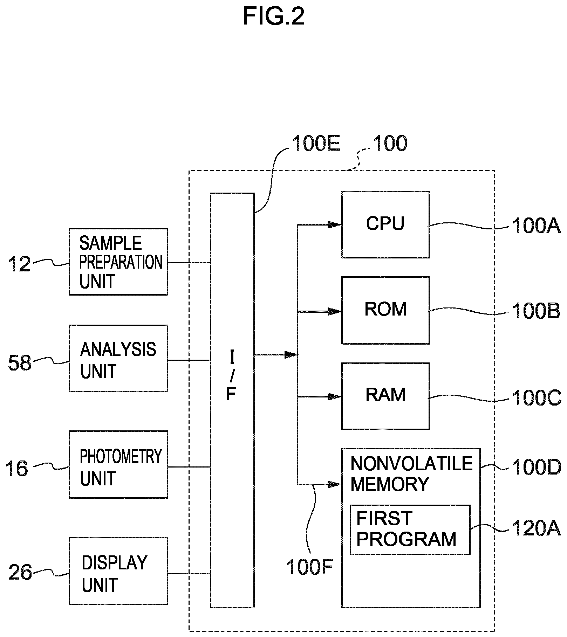

Fig. 2 is a block diagram of a control system of the HPLC apparatus 10. As illustrated in Fig. 2, the HPLC apparatus 10 includes the control unit 100. The control unit 100 has a configuration in which a central processing unit (CPU) 100A, a read only memory (ROM) 100B, a random access memory (RAM) 100C, a nonvolatile memory 100D, and an input/output interface (I/F) 100E are connected via a bus 100F. The HPLC apparatus 10 also includes an operation portion (not illustrated) that receives an input from an operator and a display unit 26.

The sample preparation unit 12, the analysis unit 58, the photometry unit 16, and the display unit 26 are connected to the input/output interface 100E.

In the present embodiment, the display unit 26 is, for example, a liquid crystal display, and is controlled by the CPU 100A to display information of the control unit 100 to the operator.

In the present embodiment, as an example, in the CPU 100A, a basic program (not illustrated) for controlling an operation of the HPLC apparatus 10 is recorded in advance in a nonvolatile memory 100D. In the present embodiment, as an example, the nonvolatile memory 100D further stores a first program 120A for generating a trained model 120C. The CPU 100A reads and executes various programs stored in the nonvolatile memory 100D. The first program 120A may be recorded on a recording medium such as a CD-ROM, and the CPU 100A may execute the first program 120A by reading the first program 120A with a CD-ROM drive or the like.

The CPU 100A controls the manifold 61 and the injection valve 63 by executing the basic program to change the type of an eluent introduced into the analytical column 60 or a flow rate of the eluent per unit time. As a result, the CPU 100A determines a flow rate of a component in the sample flowing out from the analytical column 60 per unit time, and sets a measurement time of the component contained in the sample (a time for measuring the absorbance in the photometry unit 16).

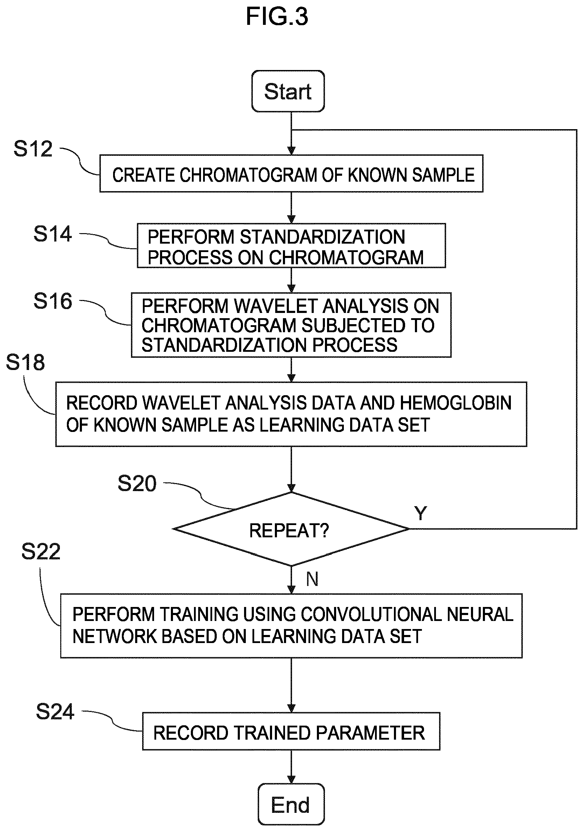

Next, a procedure of generating a trained model used for the hemoglobin species determination method according to the present embodiment will be described with reference to Figs. 3 to 6 as appropriate.

### (Procedure of Generating Trained Model according to Method of Present Embodiment)

In the trained model generation apparatus of the present embodiment, the control unit 100 executes various programs according to the present embodiment to generate a trained model for determining the species of hemoglobin contained in a sample. Specifically, in a case in which the first program 120A is read, the CPU 100A executes the following processing.

### (Procedure that First Program 120A Causes CPU 100A to Execute)

In the present embodiment, the first program 120A causes the CPU 100A to execute processing illustrated in Fig. 3.

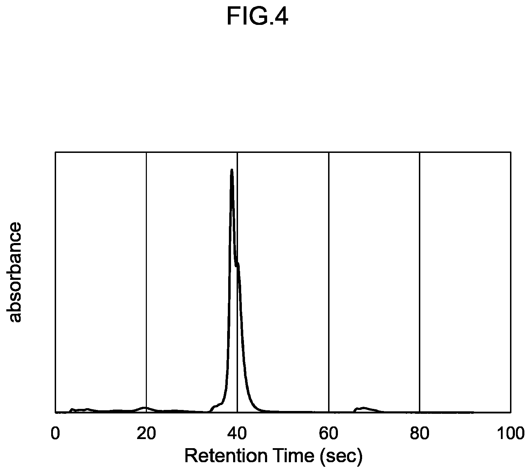

In step S12, the CPU 100A stores, in the RAM 100C, the absorbance corresponding to a component of a sample (hereinafter, referred to as a known sample) in which the species of hemoglobin is known, measured by the photometry unit 16, and creates a chromatogram as illustrated in Fig. 4. The CPU 100A stores the created chromatogram in the RAM 100C. Thereafter, the CPU 100A proceeds to step S14.

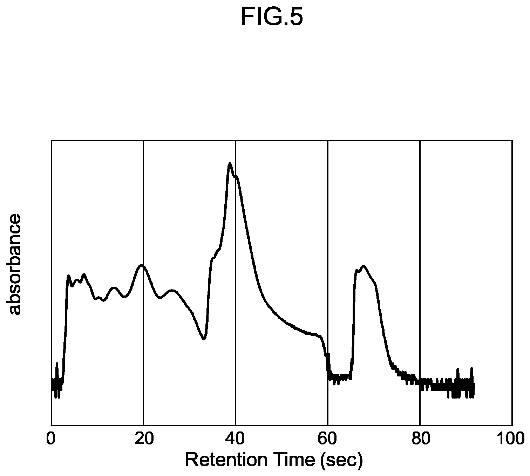

In step S14, the CPU 100A performs a standardization process on the chromatogram stored in the RAM 100C to create standardized data as illustrated in Fig. 5. The CPU 100A stores the created standardized data in the RAM 100C. Thereafter, the CPU 100A proceeds to step S16.

A specific conversion method of the standardization process is as illustrated in the following procedure and Formula 1.
1. Standardization process (a scaling process in which a value obtained by subtracting an average value from a measured value is further divided by a standard deviation for each of measured data points) is performed on each data point of the chromatogram.
2. A value obtained by adding 0.001 to an absolute value of the minimum value in the data after the standardization process is added to each data point after the standardization process.
3. For each data point, a logarithm having e (Napier's constant) as a base is taken. $x {′}_{i} = ln \left(\frac{{x}_{i} - μ}{σ}+\left|Min\left(\frac{{x}_{i} - μ}{σ}\right)\right|+0.001\right)$

In above Formula 1, x'ᵢ is a value at each data point i after the standardization process, and xᵢ is a value at each data point i in the chromatogram. µ is an average value of the data points in the chromatogram, and σ is a standard deviation of values of the data points in the chromatogram. Min is a function indicating a minimum value among a plurality of values. ln is a logarithmic function having the Napier's constant as a base (i.e. a natural logarithm).

The conversion method and the parameters described above are examples of the standardization process according to the present embodiment, and the standardization process according to the present embodiment is not limited to the conversion method and the parameters described above.

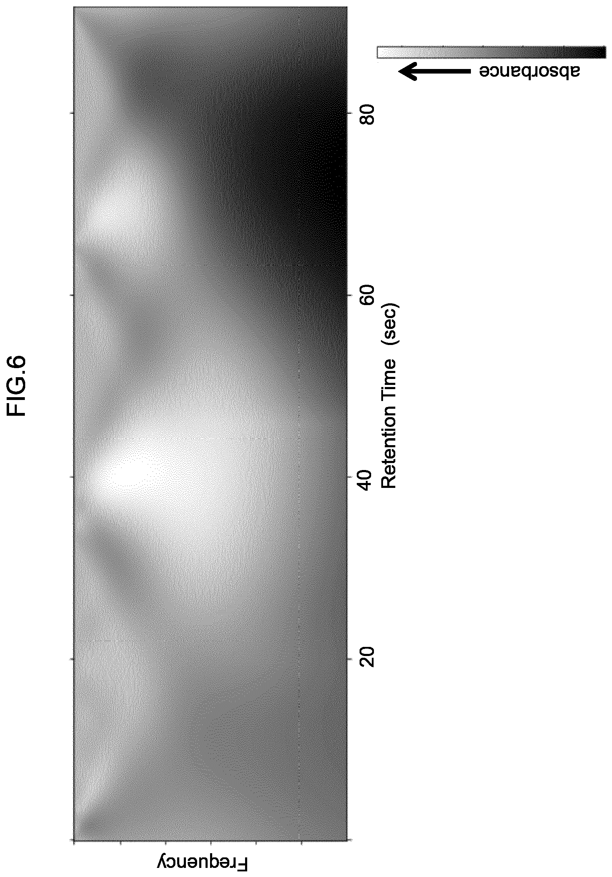

In step S16, the CPU 100A performs wavelet analysis (i.e. wavelet transform) on the standardized data stored in the RAM 100C to generate wavelet analysis data as illustrated in Fig. 6. The CPU 100A records the created wavelet analysis data in the ROM 100B. Thereafter, the CPU 100A proceeds to step S18.

In step S18, the CPU 100A creates a learning data set in which the species of hemoglobin contained in a sample from which the wavelet analysis data is created is associated with the wavelet analysis data. The CPU 100A records the created learning data set in the ROM 100B. Thereafter, the CPU 100A proceeds to step S20.

In step S20, the CPU 100A checks the number of recorded learning data sets. In a case in which the number of learning data sets is smaller than a predetermined number, an affirmative determination is made in step S20, and the processing proceeds to step S12. In a case in which the number of learning data sets has reached the predetermined number, a negative determination is made in step S20, and the processing proceeds to step S22.

In step S22, the CPU 100A constructs a convolutional neural network, and inputs the convolutional neural network as a plurality of learning data sets recorded in the ROM 100B. The CPU 100A learns features of the species and an amount of hemoglobin contained in the sample and the wavelet analysis data based on the plurality of learning data sets input to the convolutional neural network. Thereafter, the CPU 100A proceeds to step S24.

In step S24, the CPU 100A records the learned parameters in the nonvolatile memory 100D. Thereafter, the CPU 100A ends the processing using the first program 120A.

The learned parameters are a part of a trained model 120C that will be described later combined with a subprogram (not illustrated). In other words, the first program 120A causes the CPU 100A to execute processing of generating the trained model 120C.

As described above, the first program 120A performs wavelet analysis on the standardized data. As described above, in the present embodiment, the CPU 100A that executes the first program 120A may be said to be an example of a frequency analysis unit.

Next, a procedure of determining the species of hemoglobin contained in a sample (hereinafter, referred to as an unknown sample) in which the species of hemoglobin is unknown by using the trained model according to the present embodiment will be described with reference to Figs. 1, 7, and 8. In the HPLC apparatus 11 which is the hemoglobin determination apparatus according to the present embodiment, other configurations of the HPLC apparatus 10 except for the control unit 100 are similar to those of the HPLC apparatus 10.

### (Configuration of Hemoglobin Determination Apparatus)

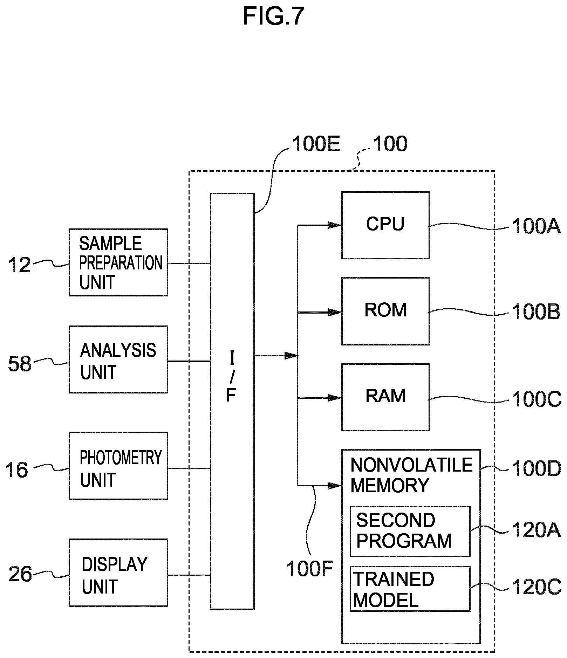

Fig. 7 is a configuration diagram of the control unit 101 of the HPLC apparatus 11 that is a hemoglobin determination apparatus according to the present embodiment. The hemoglobin determination apparatus is an example of a hemoglobin analysis system.

The HPLC apparatus 11 that is a hemoglobin determination apparatus differs from the HPLC apparatus 10 that is a trained model generation apparatus described above in a configuration of a program included in the nonvolatile memory 100D. Specifically, in the hemoglobin determination apparatus, a trained model 120C and a second program 120B for determining hemoglobin in a blood sample by using the trained model 120C are stored in the nonvolatile memory.

The CPU 100A in the hemoglobin determination apparatus reads and executes various programs stored in the nonvolatile memory 100D. The trained model 120C includes learned parameters generated by the trained model generation apparatus described above. The trained model 120C may be transferred in any manner from the nonvolatile memory 100D of the trained model generation apparatus, and is recorded in the nonvolatile memory 100D of the hemoglobin determination apparatus via the network as an example. The second program 120B may be recorded on a recording medium such as a CD-ROM, and the CPU 100A may execute the second program 120B by reading the second program 120B with a CD-ROM drive or the like.

Other configurations of the control unit 101 are similar to those of the control unit 100 of the HPLC apparatus 10 that is the trained model generation apparatus described above. In the following description, in a case in which the HPLC apparatus 10 that is the trained model generation apparatus and the HPLC apparatus 11 that is the hemoglobin determination apparatus are not distinguished from each other, the apparatuses will be referred to as HPLC apparatuses 10 and 11. Similarly, in the following description, in a case in which the control unit 100 of the HPLC apparatus 10 and the control unit 101 of the HPLC apparatus 11 are not distinguished from each other, the units will be referred to as control units 100 and 101.

### (Procedure of Determining Type of Hemoglobin according to Method of Present Embodiment)

In the hemoglobin determination apparatus of the present embodiment, the control unit 101 executes various programs according to the present embodiment to determine the species of hemoglobin contained in a sample. Specifically, in a case in which the second program 120B is read, the CPU 100A executes the following processing.

### (Processing that Second Program 120B Causes CPU 100A to Execute)

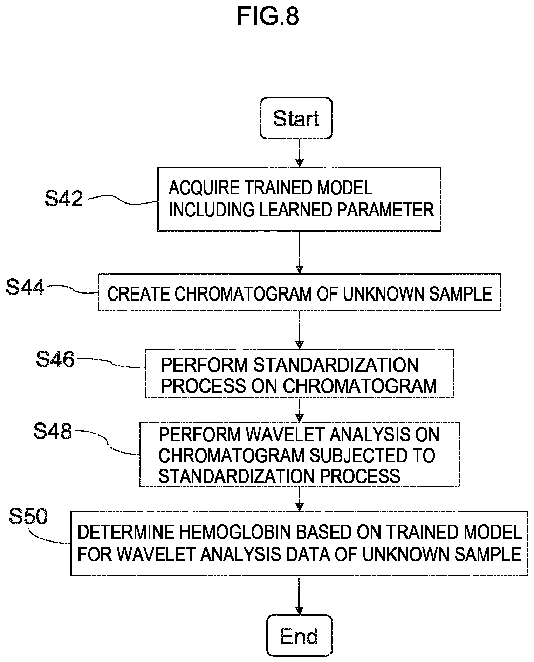

In the present embodiment, the second program 120B causes the CPU 100A to execute processing illustrated in Fig. 8.

In step S42, the CPU 100A reads the trained model 120C (learned parameter and subprogram) recorded in the ROM 100B. Thereafter, the CPU 100A proceeds to step S44.

In step S44, the CPU 100A stores the absorbance corresponding to a component of an unknown sample measured by the photometry unit 16 in the RAM 100C, and creates a chromatogram as illustrated in Fig. 4. The CPU 100A stores the created chromatogram in the RAM 100C. Thereafter, the CPU 100A proceeds to step S46.

In step S46, the CPU 100A performs a standardization process on the chromatogram stored in the RAM 100C, and creates standardized data as illustrated in Fig. 5. The CPU 100A stores the created standardized data in the RAM 100C. Thereafter, the CPU 100A proceeds to step S48.

In step S48, the CPU 100A performs wavelet analysis on the standardized data stored in the RAM 100C to generate wavelet analysis data as illustrated in Fig. 6. The CPU 100A records the created wavelet analysis data in the ROM 100B.

In step S50, the CPU 100A inputs the wavelet analysis data recorded in the ROM 100B to the trained model 120C, and outputs the species of hemoglobin contained in the unknown sample. Thereafter, the CPU 100A ends the processing using the second program 120B.

The hemoglobin determination apparatus according to the present embodiment may further execute the following processing.

For example, in step S50, in a case in which the species of hemoglobin contained in the unknown sample is not determined, the CPU 100A may display, on the display unit 26, the fact that the species of hemoglobin is not determined.

In step S50, in a case in which the species of hemoglobin contained in the unknown sample is determined, another process may be further performed. For example, the CPU 100A may calculate an amount of hemoglobin contained in the unknown sample for each hemoglobin species from a value of a peak corresponding to the hemoglobin species that is a target of the chromatogram of the unknown sample, and display the calculated value on the display unit 26.

As described above, the second program 120B performs wavelet analysis on the standardized data. As described above, in the present embodiment, the CPU 100A that executes the second program 120B may be said to be an example of a frequency analysis unit.

Next, a result of determining hemoglobin contained in an unknown sample by using the method of generating the trained model 120C and the hemoglobin species determination method according to the present embodiment will be described as Example 1 and Example 2. In each example, the "unknown sample" is a sample used for a determination test in the trained model 120C, and is a sample not used for generation of the trained model 120C in which the species of hemoglobin contained in the sample is actually determined.

In each example, the diabetes tester HA-8180V or HA-8190V manufactured by ARKRAY Inc. was used. In each measurement device, the measurement time was set to 90 seconds for HA-8180V and 24 seconds for HA-8190V. A sampling frequency was 2048 Hz (sampling interval: 488.28125 microseconds).

In each example, a blood sample containing hemoglobin S, hemoglobin C, hemoglobin D, or hemoglobin E and a blood sample in which hemoglobin S, hemoglobin C, hemoglobin D, and hemoglobin E were not observed were prepared. Hereinafter, a sample in which hemoglobin S, hemoglobin C, hemoglobin D, and hemoglobin E are not observed will be referred to as a "healthy subject specimen".

### (Example 1)

In Example 1, the trained model 120C was created by using HA-8180V as a measurement device, and the species of hemoglobin contained in the unknown sample was determined by using the trained model 120C.

In the present example, learning was performed by using the procedure illustrated in Fig. 3 in the learning stage. More specifically, in the present example, a chromatogram of a known sample was created, and the created chromatogram was subjected to a standardization process, and then wavelet analysis data was created. A learning data set including the wavelet analysis data was created, and learning was performed by a convolutional neural network to create the trained model 120C.

In the present example, a determination is performed by using the procedure illustrated in Fig. 8 in the unknown sample estimation stage. More specifically, in the present example, a chromatogram of an unknown sample was created, and the created chromatogram was subjected to a standardization process, and then wavelet analysis data was created. The species of hemoglobin contained in the unknown sample was determined based on the trained model 120C for the wavelet analysis data.

Table 1 illustrates the number of pieces of learning data (which is the number of learning data sets and is the number of known samples), the number of pieces of test data (which is the number of samples used in the determination test and is the number of unknown samples), the number of correct answers, and a correct answer ratio used for learning according to the present example.

**[Table 1]**

| | Number of pieces of learning data | Number of pieces of test data | Number of correct answers | Ratio of correct answers |
|---|---|---|---|---|
| Healthy subject specimen | 677 | 163 | 160 | 0.982 |
| Hemoglobin S | 730 | 181 | 181 | 1.000 |
| Hemoglobin C | 563 | 158 | 158 | 1.000 |
| Hemoglobin D | 651 | 163 | 162 | 0.994 |
| Hemoglobin E | 743 | 186 | 179 | 0.962 |
| Others | 230 | 126 | 100 | 0.794 |
| Total | 3594 | 977 | 940 | 0.962 |

### (Example 2)

**In** Example 2, the trained model 120C was created by using HA-8190V as a measurement device, and the species of hemoglobin contained in the unknown sample was determined by using the trained model 120C.

**In** the present example, learning was performed by using the procedure illustrated in Fig. 3 in the learning stage. More specifically, in the present example, a chromatogram of a known sample was created, and the created chromatogram was subjected to a standardization process, and then wavelet analysis data was created. A learning data set including wavelet analysis data was created, and learning was performed by a convolutional neural network to create the trained model.

**In the** present example, a determination is performed by using the procedure illustrated in Fig. 8 in the unknown sample estimation stage. More specifically, in the present example, a chromatogram of an unknown sample was created, and the created chromatogram was subjected to a standardization process, and then wavelet analysis data was created. The species of hemoglobin contained in the unknown sample was determined based on the trained model for the wavelet analysis data.

Table 2 illustrates the number of pieces of learning data, the number of pieces of test data, the number of correct answers, and a correct answer ratio used for learning according to the present example.

**[Table 2]**

| | Number of pieces of learning data | Number of pieces of test data | Number of correct answers | Ratio of correct answers |
|---|---|---|---|---|
| Healthy subject specimen | 173 | 43 | 43 | 1.000 |
| Hemoglobin S | 124 | 31 | 30 | 0.968 |
| Hemoglobin C | 98 | 25 | 25 | 1.000 |
| Hemoglobin D | 78 | 20 | 12 | 0.600 |
| Hemoglobin E | 396 | 99 | 99 | 1.000 |
| Others | 324 | 81 | 77 | 0.951 |
| Total | 1193 | 299 | 286 | 0.957 |

### (Summary)

As described above, the following content was shown by the HPLC apparatuses 10 and 11 in the present embodiment.

As illustrated in Tables 1 and 2, it was shown that the species of hemoglobin can be determined by using the method of creating a trained model and the hemoglobin species determination method in Example 1 and Example 2. From this result, it is considered that the accuracy of specifying the species of hemoglobin in a blood sample based on the trained model created by using the wavelet analysis data is improved. That is, it was shown that the species of hemoglobin in the blood sample can be specified even in a case in which the separation time is short according to the method of creating a trained model and the hemoglobin species determination method in the present embodiment.

### (Method of Determining Type of Hemoglobin in Reference Example)

Incidentally, as a method of determining a species of hemoglobin contained in an unknown sample, there is a hemoglobin species determination method based on a shape of a peak appearing in a chromatogram (a positional relationship between peaks appearing in the chromatogram).

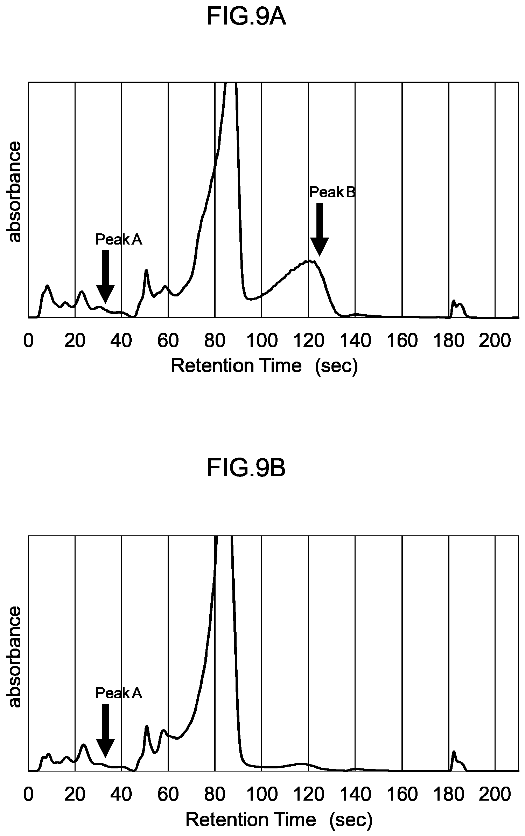

Fig. 9A is a chromatogram acquired for a blood sample containing hemoglobin E under the condition of using HA-8180V as a measurement device and setting a flow rate so that a measurement time is 200 seconds. As illustrated in Fig. 9A, in this sample, two peaks were confirmed before and after the largest peak which is a peak of hemoglobin A0.

Similarly, Fig. 9B is a chromatogram acquired under the condition that a measurement time is set to 200 seconds for a blood sample containing abnormal hemoglobin A. As illustrated in Fig. 9B, according to this chromatogram, a peak A appeared around 30 seconds as a fractionated region, but unlike Fig. 9A, a peak B did not appear. From this, according to this measurement condition, it is considered that it is possible to determine between hemoglobin E and abnormal hemoglobin A by observing the presence or absence of the peak B for the sample having the peak A. As described above, hemoglobin can be determined by setting the measurement time to 200 seconds, but this measurement time is longer than the time set in the above Example 1.

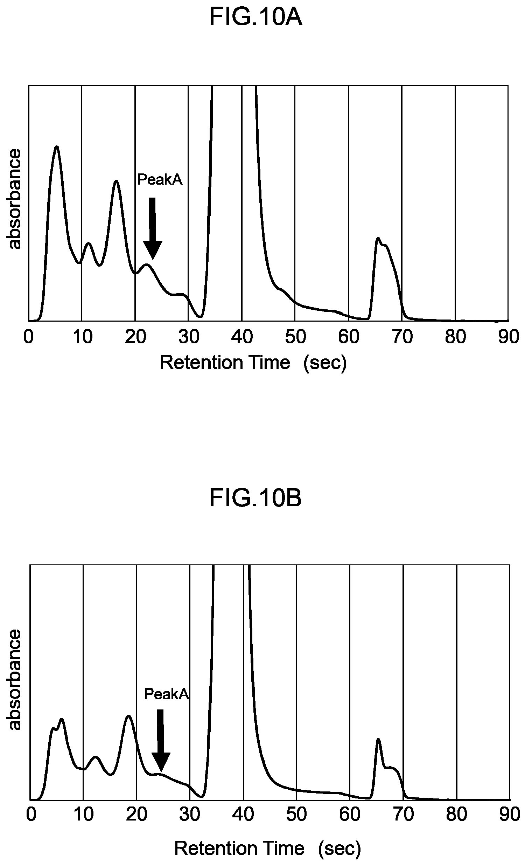

Here, Fig. 10A is a chromatogram acquired by using the same sample and the same apparatus as in Fig. 9A under the condition that the measurement time is set to 90 seconds as in Example 1. As illustrated in Fig. 10A, according to this chromatogram, the peak A appeared around 25 seconds as a fractionated region, but the peak B did not appear.

Similarly, Fig. 10B is a chromatogram acquired by using the same sample as that in Fig. 9B and HA-8190V that is a different measurement device under the condition that the measurement time is set to 90 seconds. As illustrated in Fig. 10B, according to this chromatogram, the peak A appeared around 25 seconds as a fractionated region, but the peak B did not appear.

As described above, since the peak B occurring in Fig. 9A is not fractionated in Fig. 10A, in a case in which the measurement time is shortened, the species of hemoglobin contained in the sample is likely to be erroneously determined.

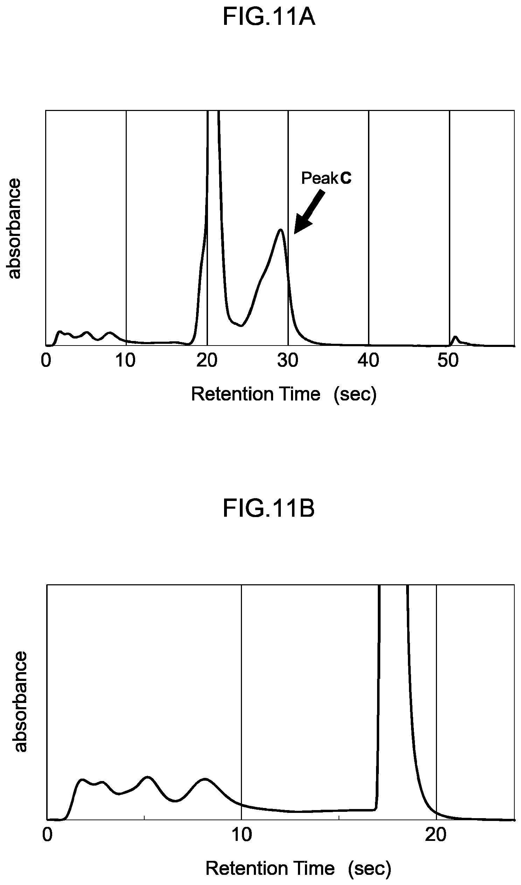

For example, Fig. 11A is a chromatogram acquired under the condition that a measurement time is set to 90 seconds for a blood sample containing hemoglobin S. As illustrated in Fig. 11A, in the sample containing hemoglobin S, a peak C appeared around 28 seconds, which is after the largest peak (which is the peak of hemoglobin A0) appeared around 20 seconds.

Fig. 11B is a chromatogram acquired by using the same sample and the same apparatus as in Fig. 11A under the condition that the measurement time is set to 24 seconds as in Example 2. As illustrated in Fig. 11B, according to this chromatogram, the peak C did not appear after the largest peak appearing around 18 seconds.

As described above, even in the case of using the same HPLC apparatuses 10 and 11, in a case in which the measurement time is shortened, the species of hemoglobin contained in a sample is likely to be erroneously determined.

In HA-8180V that is an example of the HPLC apparatuses 10 and 11 in the present embodiment, it is shown that the species of hemoglobin in a blood sample can be specified during a measurement time of 90 seconds. In HA-8190V that is an example of the HPLC apparatuses 10 and 11 in the present embodiment, it is shown that the species of hemoglobin in a blood sample can be specified during a measurement time of 24 seconds. Therefore, it was shown that the trained model 120C is created by using the wavelet analysis data, and thus the species of hemoglobin in the blood sample can be specified even in a case in which separation data having a short separation time of a separation process on the blood sample is used.

Even in a case in which the separation time of the separation process on the blood sample is long, a peak value of the chromatogram is not clear, and the determination accuracy of the species of hemoglobin based on the chromatogram may deteriorate. In the trained model generation method and the hemoglobin species determination method according to the present embodiment, since the frequency analysis is performed on a component contained in a blood sample, even if peaks overlap and a peak appearing in the chromatogram is somewhat unclear, the overlapping peaks are separated as a frequency analysis result. Therefore, in the trained model generation method and the hemoglobin species determination method according to the present embodiment, an effect of improving a determination system for the species of hemoglobin can be expected even in a chromatogram in which a peak value is not clear.

For hemoglobin F, hemoglobin A2 (HbA2), and other mutant hemoglobin species not implemented in Example 1 and Example 2 described above, similarly to the present embodiment, a method of generating trained model and determining the species of hemoglobin can be applied.

### (Other Embodiments)

In the above description, the control units 100 and 101 are incorporated in the HPLC apparatuses 10 and 11, but the present invention is not limited thereto. For example, main constituents of the control units 100 and 101 may be disposed on a network, and the HPLC apparatuses 10 and 11 may be operated as the control units 100 and 101 through communication with the constituents disposed on the network. For example, the first program 120A and the second program 120B may be acquired by being downloaded as appropriate by the control units 100 and 101 of the HPLC apparatuses 10 and 11 from a server disposed on the network. For example, after a server disposed on a network communicates with the HPLC apparatuses 10 and 11 and the HPLC apparatuses 10 and 11 transmit chromatograms to the server, the server may transmit frequency analysis data to the HPLC apparatuses 10 and 11 to be operated as a frequency analysis unit.

The first program 120A, the second program 120B, and the trained model 120C described above are executed by the control units 100 and 101, but are not limited thereto, and may be executed by a server disposed on a network. For example, the HPLC apparatuses 10 and 11 may be configured to transmit a chromatogram of an unknown sample to a server disposed on a network and receive an amount of hemoglobin contained in the unknown sample from the server. Any of these configurations is included in the hemoglobin analysis system in the present embodiment.

In the hemoglobin species determination method according to the invention, the method of preparing the trained model 120C is not limited as long as the trained model 120C is prepared in the hemoglobin species determination method according to the invention. In other words, as long as the trained model 120C is prepared, a device that creates a chromatogram, a device that performs frequency analysis, a device that generates the trained model 120C, and a device that performs a determination are not limited.

More specifically, the device that creates a chromatogram may be a device that is separate from the HPLC apparatuses 10 and 11 and has only a function of creating a chromatogram from a blood sample. The device that performs frequency analysis may be a device that is separate from the HPLC apparatuses 10 and 11 and has only a function of performing only wavelet analysis from a chromatogram. In the above description, the trained model generation apparatus generates the trained model 120C, and the hemoglobin determination apparatus determines the species of hemoglobin based on the trained model 120C. However, these devices may be the same device. These devices may be incorporated into the same HPLC apparatuses 10 and 11, some devices may be incorporated into a common device, or each may be a separate device. Therefore, according to the technology according to the present embodiment, as long as the trained model 120C created for each model of the HPLC apparatus 11 is prepared, it is not necessary to create the trained model 120C in each individual HPLC apparatus 10.

In the above description, a case where the processing according to the embodiment is realized by a software configuration by the control units 100 and 101 executing various programs has been described, but the invention is not limited thereto. For example, the processing according to the above embodiments may be executed by a hardware configuration or a combination of a hardware configuration and a software configuration.

According to the invention, the wavelet analysis data is used as frequency analysis data.

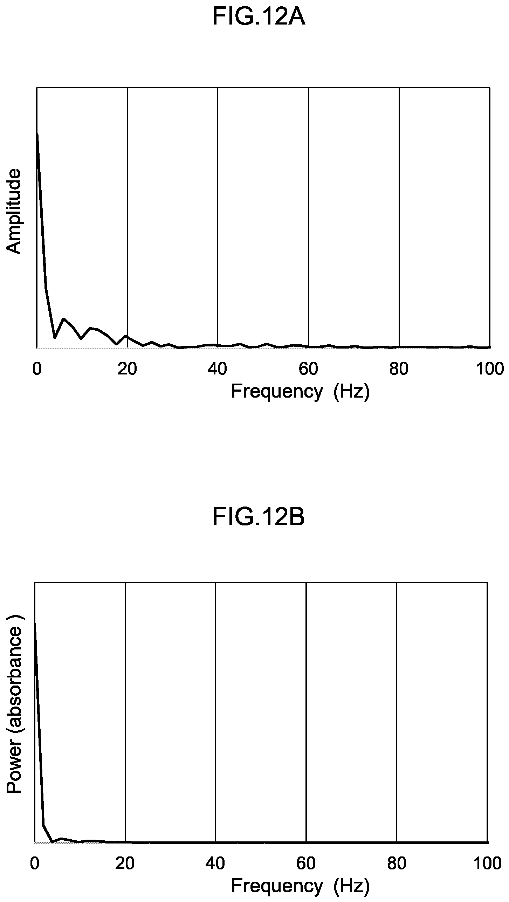

According to a non-claimed example, in the frequency analysis, by performing fast Fourier transform instead of the wavelet analysis, amplitude data as illustrated in Fig. 12A or power spectrum data as illustrated in Fig. 12B may be used. Instead of the wavelet analysis, data subjected to short-time Fourier transform may be used.

**In** the above description, a hemoglobin concentration and wavelet analysis data of a known sample are used as a learning data set, but the invention is not limited thereto, and a chromatogram may be included in a learning data set. **In** this case, in the determination procedure, the chromatogram of the unknown sample is included in data to be input to the trained model 120C.

In the above description, the HPLC apparatuses 10 and 11 are used as an example of the hemoglobin analysis system, but the technology according to the invention is not limited thereto. That is, as long as chromatograms of known samples and unknown samples can be obtained, not only the HPLC apparatuses 10 and 11 but also an electrophoresis analyzer may be used. In the electrophoretic analyzer, a standardization process may be freely performed based on a generated pherogram, and wavelet analysis may be performed to generate wavelet analysis data. According to the technology of the invention, also in this case, it is possible to generate the trained model 120C and determine the species of hemoglobin.

In the above description, in the method of generation the trained model 120C, the trained model 120C is generated after constructing a convolutional neural network, but the technology according to the invention is not limited thereto. That is, as long as the trained model 120C is obtained, for example, a recurrent neural network may be constructed and trained. Also in this case, the trained model 120C can be generated.

## Claims

1. A hemoglobin species determination method, comprising:
preparing, using a computer (100), a trained model generated by using, as a learning data set for machine learning, a data set including learning frequency analysis data generated by performing frequency analysis on separation data generated by a separation process on a learning blood sample in which a species of hemoglobin is known and including the known species of hemoglobin;
preparing, using a computer, test frequency analysis data by performing frequency analysis on separation data generated by a separation process on a test blood sample in which the species of hemoglobin is unknown; and
determining, using a computer, the species of hemoglobin contained in the test blood sample by inputting the test frequency analysis data to the trained model,
wherein:
the separation data for the learning frequency analysis data and the separation data for the test frequency analysis data are chromatograms;
the learning frequency analysis data and the test frequency analysis data are generated by performing frequency analysis on the separation data for the learning frequency analysis data and the separation data for the test frequency analysis data, the separation data for the learning frequency analysis data and the separation data for the test frequency analysis data having been subjected to a standardization process; and
the learning frequency analysis data and the test frequency analysis data are generated via wavelet analysis.

2. The hemoglobin species determination method according to claim 1, wherein:
the machine learning includes a convolutional neural network.

3. The hemoglobin species determination method according to any preceding claim, wherein:
the hemoglobin is hemoglobin S, hemoglobin C, hemoglobin D, or hemoglobin E.

4. A trained model generation method, comprising:
generating a learned parameter for a trained model configured to determine a species of hemoglobin contained in a blood sample by using, as a learning data set for machine learning, a data set including learning frequency analysis data generated by performing frequency analysis on separation data generated by a separation process on a learning blood sample in which a species of hemoglobin is known and including the known species of hemoglobin;
wherein:
the separation data is a chromatogram;
the frequency analysis for generating the learning frequency analysis data is performed on separation data subjected to a standardization process; and
the frequency analysis for generating the learning frequency analysis data is wavelet analysis.

5. The trained model generation method according to claim 4, wherein:
the machine learning includes a convolutional neural network.

6. The trained model generation method according to claims 4 or 5, wherein:
the hemoglobin is hemoglobin S, hemoglobin C, hemoglobin **D,** or hemoglobin E.

7. A hemoglobin analysis system, comprising:
a separation unit (16, 58) that performs a separation process on a test blood sample in which a species of hemoglobin is unknown and generates test separation data;
a frequency analysis unit (100A) that performs frequency analysis on the test separation data and generates test frequency analysis data;
a determination unit that determines the species of hemoglobin contained in the test blood sample by using a trained model (120C), the species of hemoglobin being measured by the test frequency analysis data; and
an output unit that outputs the species of hemoglobin determined by the determination unit;
wherein:
the trained model outputs a species of hemoglobin contained in a blood sample from frequency analysis data in a case in which the frequency analysis data generated by performing frequency analysis on separation data generated by a separation process on the blood sample is input, wherein:
the trained model is generated by using, as a learning data set for machine learning, learning data including learning frequency analysis data generated by performing frequency analysis on separation data generated by a separation process on a learning blood sample in which the species of hemoglobin is known, and the known species of hemoglobin,
the separation data for the learning frequency analysis data and the separation data for the test frequency analysis data are chromatograms;
the learning frequency analysis data and the test frequency analysis data are generated by performing frequency analysis on the separation data for the learning frequency analysis data and the separation data for the test frequency analysis data, the separation data for the learning frequency analysis data and the separation data for the test frequency analysis data having been subjected to a standardization process; and
the learning frequency analysis data and the test frequency analysis data are generated via wavelet analysis.

8. An information processing program executable by a computer (100) to perform processing, the processing comprising:
generating a trained model by using, as a learning data set of machine learning, learning data including learning frequency analysis data generated by performing frequency analysis on separation data generated by a separation process on a learning blood sample in which a species of hemoglobin is known, and the known species of hemoglobin;
receiving test frequency analysis data generated by performing frequency analysis on separation data generated by a separation process on a test blood sample in which the species of hemoglobin is unknown; and
outputting the species of hemoglobin contained in the test blood sample based on the trained model,
wherein:
the separation data for the learning frequency analysis data and the separation data for the test frequency analysis data are chromatograms;
the learning frequency analysis data and the test frequency analysis data are generated by performing frequency analysis on the separation data for the learning frequency analysis data and the separation data for the test frequency analysis data, the separation data for the learning frequency analysis data and the separation data for the test frequency analysis data having been subjected to a standardization process; and
the learning frequency analysis data and the test frequency analysis data are generated via wavelet analysis.

## Patentansprüche

1. Hämoglobinart-Bestimmungsverfahren, umfassend:
Herstellen, unter Verwendung eines Computers (100), eines trainierten Modells, das durch Verwenden, als Lerndatensatz zum Maschinenlernen, eines Datensatzes erzeugt wird, der Lernfrequenzanalysedaten einschließt, die durch Durchführen einer Frequenzanalyse an Trenndaten erzeugt werden, die durch einen Trennprozess an einer Lernblutprobe erzeugt werden, in der eine Hämoglobinart bekannt ist und die die bekannte Hämoglobinart einschließt;
Herstellen, unter Verwendung eines Computers, von Testfrequenzanalysedaten durch Durchführen einer Frequenzanalyse an Trenndaten, die durch einen Trennprozess an einer Testblutprobe erzeugt werden, in der die Hämoglobinart unbekannt ist; und
Bestimmen, unter Verwendung eines Computers, der Hämoglobinart, die in der Testblutprobe enthalten ist, durch Eingeben der Testfrequenzanalysedaten in das trainierte Modell,
wobei:
die Trenndaten für die Lernfrequenzanalysedaten und die Trenndaten für die Testfrequenzanalysedaten Chromatogramme sind;
die Lernfrequenzanalysedaten und die Testfrequenzanalysedaten erzeugt werden, indem eine Frequenzanalyse an den Trenndaten für die Lernfrequenzanalysedaten und den Trenndaten für die Testfrequenzanalysedaten durchgeführt wird, wobei die Trenndaten für die Lernfrequenzanalysedaten und die Trenndaten für die Testfrequenzanalysedaten einem Standardisierungsprozess unterworfen wurden; und
die Lernfrequenzanalysedaten und die Testfrequenzanalysedaten durch Wavelet-Analyse erzeugt werden.

2. Hämoglobinart-Bestimmungsverfahren nach Anspruch 1, wobei:
das Maschinenlernen ein gefaltetes neuronales Netzwerk einschließt.

3. Hämoglobinart-Bestimmungsverfahren nach einem vorstehenden Anspruch, wobei:
das Hämoglobin Hämoglobin S, Hämoglobin C, Hämoglobin D oder Hämoglobin E ist.

4. Verfahren zur Erzeugung eines trainierten Modells, umfassend:
Erzeugen eines erlernten Parameters für ein trainiertes Modell, das ausgebildet ist, um eine Hämoglobinart zu bestimmen, die in einer Blutprobe enthalten ist, durch Verwenden, als Lerndatensatz zum Maschinenlernen, eines Datensatzes, der Lernfrequenzanalysedaten einschließt, die durch Durchführen einer Frequenzanalyse an Trenndaten erzeugt werden, die durch einen Trennprozess an einer Lernblutprobe erzeugt werden, in der eine Hämoglobinart bekannt ist und die die bekannte Hämoglobinart einschließt;
wobei:
die Trenndaten ein Chromatogramm sind;
die Frequenzanalyse zum Erzeugen der Lernfrequenzanalysedaten an Trenndaten durchgeführt wird, die einem Standardisierungsprozess unterworfen werden; und
die Frequenzanalyse zum Erzeugen der Lernfrequenzanalysedaten eine Wavelet-Analyse ist.

5. Verfahren zur Erzeugung eines trainierten Modells nach Anspruch 4, wobei:
das Maschinenlernen ein gefaltetes neuronales Netzwerk einschließt.

6. Verfahren zur Erzeugung eines trainierten Modells nach Anspruch 4 oder 5, wobei:
das Hämoglobin Hämoglobin S, Hämoglobin C, Hämoglobin D oder Hämoglobin E ist.

7. Hämoglobinanalysesystem, umfassend:
eine Trenneinheit (16, 58), die einen Trennprozess an einer Testblutprobe durchführt, in der eine Hämoglobinart unbekannt ist und die Testtrenndaten erzeugt;
eine Frequenzanalyseeinheit (100A), die eine Frequenzanalyse an den Testtrenndaten durchführt und Testfrequenzanalysedaten erzeugt;
eine Bestimmungseinheit, die die Hämoglobinart bestimmt, die in der Testblutprobe enthalten ist, durch Verwenden eines trainierten Modells (120C), wobei die Hämoglobinart durch die Testfrequenzanalysedaten gemessen wird; und
eine Ausgabeeinheit, die die von der Bestimmungseinheit bestimmte Hämoglobinart ausgibt;
wobei:
das trainierte Modell eine Hämoglobinart, die in einer Blutprobe enthalten ist, aus Frequenzanalysedaten in einem Fall ausgibt, bei dem die Frequenzanalysedaten, die durch Durchführen einer Frequenzanalyse an Trenndaten erzeugt werden, die durch einen Trennprozess in der Blutprobe erzeugt werden, eingegeben werden, wobei:
das trainierte Modell erzeugt wird, indem als Lerndatensatz zum Maschinenlernen Lerndaten verwendet werden, die Lernfrequenzanalysedaten einschließen, die durch Durchführen einer Frequenzanalyse an Trenndaten erzeugt werden, die durch einen Trennprozess an einer Lernblutprobe erzeugt werden, in der die Hämoglobinart bekannt ist und die die bekannte Hämoglobinart einschließt;
die Trenndaten für die Lernfrequenzanalysedaten und die Trenndaten für die Testfrequenzanalysedaten Chromatogramme sind;
die Lernfrequenzanalysedaten und die Testfrequenzanalysedaten erzeugt werden, indem eine Frequenzanalyse an den Trenndaten für die Lernfrequenzanalysedaten und den Trenndaten für die Testfrequenzanalysedaten durchgeführt wird, wobei die Trenndaten für die Lernfrequenzanalysedaten und die Trenndaten für die Testfrequenzanalysedaten einem Standardisierungsprozess unterworfen wurden; und
die Lernfrequenzanalysedaten und die Testfrequenzanalysedaten durch Wavelet-Analyse erzeugt werden.

8. Informationsverarbeitungsprogramm, das von einem Computer (100) ausführbar ist, um eine Verarbeitung durchzuführen, wobei die Verarbeitung Folgendes umfasst:
Erzeugen eines trainierten Modells durch Verwenden, als Lerndatensatz zum Maschinenlernen, von Lerndaten, die Lernfrequenzanalysedaten einschließen, die durch Durchführen einer Frequenzanalyse an Trenndaten erzeugt werden, die durch einen Trennprozess an einer Lernblutprobe erzeugt werden, in der eine Hämoglobinart bekannt ist und die die bekannte Hämoglobinart einschließt;
Empfangen von Testfrequenzanalysedaten, die durch Durchführen einer Frequenzanalyse an Trenndaten erzeugt werden, die durch einen Trennprozess an einer Testblutprobe erzeugt werden, in der die Hämoglobinart unbekannt ist; und
Ausgeben der in der Testblutprobe enthaltenen Hämoglobinart auf der Grundlage des trainierten Modells,
wobei:
die Trenndaten für die Lernfrequenzanalysedaten und die Trenndaten für die Testfrequenzanalysedaten Chromatogramme sind;
die Lernfrequenzanalysedaten und die Testfrequenzanalysedaten erzeugt werden, indem eine Frequenzanalyse an den Trenndaten für die Lernfrequenzanalysedaten und den Trenndaten für die Testfrequenzanalysedaten durchgeführt wird, wobei die Trenndaten für die Lernfrequenzanalysedaten und die Trenndaten für die Testfrequenzanalysedaten einem Standardisierungsprozess unterworfen wurden; und
die Lernfrequenzanalysedaten und die Testfrequenzanalysedaten durch Wavelet-Analyse erzeugt werden.

## Revendications

1. Procédé de détermination de type d'hémoglobine, consistant à :
préparer, en utilisant un ordinateur (100), un modèle entraîné généré par l'utilisation, en tant qu'ensemble de données d'apprentissage pour l'apprentissage automatique, d'un ensemble de données incluant des données d'analyse fréquentielle d'apprentissage générées par la réalisation d'une analyse fréquentielle sur des données de séparation générées par un processus de séparation sur un échantillon de sang d'apprentissage dans lequel un type d'hémoglobine est connu et incluant le type d'hémoglobine connu ;
préparer, en utilisant un ordinateur, des données d'analyse fréquentielle de test par la réalisation d'une analyse fréquentielle sur des données de séparation générées par un processus de séparation sur un échantillon de sang de test dans lequel le type d'hémoglobine est inconnu ; et
déterminer, en utilisant un ordinateur, le type d'hémoglobine contenu dans l'échantillon de sang de test par l'entrée des données d'analyse fréquentielle de test dans le modèle entraîné,
dans lequel :
les données de séparation pour les données d'analyse fréquentielle d'apprentissage et les données de séparation pour les données d'analyse fréquentielle de test sont des chromatogrammes ;
les données d'analyse fréquentielle d'apprentissage et les données d'analyse fréquentielle de test sont générées par la réalisation d'une analyse fréquentielle sur les données de séparation pour les données d'analyse fréquentielle d'apprentissage et les données de séparation pour les données d'analyse fréquentielle de test, les données de séparation pour les données d'analyse fréquentielle d'apprentissage et les données de séparation pour les données d'analyse fréquentielle de test ayant été soumises à un processus de standardisation ; et
les données d'analyse fréquentielle d'apprentissage et les données d'analyse fréquentielle de test sont générées par le biais d'une analyse en ondelettes.

2. Procédé de détermination de type d'hémoglobine selon la revendication 1, dans lequel :
l'apprentissage automatique inclut un réseau de neurones convolutionnel.

3. Procédé de détermination de type d'hémoglobine selon une quelconque revendication précédente, dans lequel :
l'hémoglobine est l'hémoglobine S, l'hémoglobine C, l'hémoglobine D, ou l'hémoglobine E.

4. Procédé de génération de modèle entraîné, consistant à :
générer un paramètre appris pour un modèle entraîné configuré pour déterminer un type d'hémoglobine contenu dans un échantillon de sang par l'utilisation, en tant qu'ensemble de données d'apprentissage pour l'apprentissage automatique, d'un ensemble de données incluant des données d'analyse fréquentielle d'apprentissage générées par la réalisation d'une analyse fréquentielle sur des données de séparation générées par un processus de séparation sur un échantillon de sang d'apprentissage dans lequel un type d'hémoglobine est connu et incluant le type d'hémoglobine connu ;
dans lequel :
les données de séparation sont un chromatogramme ;
l'analyse fréquentielle pour générer les données d'analyse fréquentielle d'apprentissage est réalisée sur des données de séparation soumises à un processus de standardisation ; et
l'analyse fréquentielle pour générer les données d'analyse fréquentielle d'apprentissage est une analyse en ondelettes.

5. Procédé de génération de modèle entraîné selon la revendication 4, dans lequel :
l'apprentissage automatique inclut un réseau de neurones convolutionnel.

6. Procédé de génération de modèle entraîné selon les revendications 4 ou 5, dans lequel :
l'hémoglobine est l'hémoglobine S, l'hémoglobine C, l'hémoglobine D, ou l'hémoglobine E.

7. Système d'analyse d'hémoglobine, comprenant :
une unité de séparation (16, 58) qui réalise un processus de séparation sur un échantillon de sang de test dans lequel un type d'hémoglobine est inconnu et génère des données de séparation de test ;
une unité d'analyse fréquentielle (100A) qui réalise une analyse fréquentielle sur les données de séparation de test et génère des données d'analyse fréquentielle de test ;
une unité de détermination qui détermine le type d'hémoglobine contenu dans l'échantillon de sang de test par l'utilisation d'un modèle entraîné (120C), le type d'hémoglobine étant mesuré par les données d'analyse fréquentielle de test ; et
une unité de sortie qui fournit en sortie le type d'hémoglobine déterminé par l'unité de détermination ;
dans lequel :
le modèle entraîné fournit en sortie un type d'hémoglobine contenu dans un échantillon de sang à partir de données d'analyse fréquentielle dans un cas dans lequel les données d'analyse fréquentielle générées par la réalisation d'une analyse fréquentielle sur des données de séparation générées par un processus de séparation sur l'échantillon de sang sont entrées, dans lequel :
le modèle entraîné est généré par l'utilisation, en tant qu'ensemble de données d'apprentissage pour l'apprentissage automatique, de données d'apprentissage incluant des données d'analyse fréquentielle d'apprentissage générées par la réalisation d'une analyse fréquentielle sur des données de séparation générées par un processus de séparation sur un échantillon de sang d'apprentissage dans lequel le type d'hémoglobine est connu, et le type d'hémoglobine connu,
les données de séparation pour les données d'analyse fréquentielle d'apprentissage et les données de séparation pour les données d'analyse fréquentielle de test sont des chromatogrammes ;
les données d'analyse fréquentielle d'apprentissage et les données d'analyse fréquentielle de test sont générées par la réalisation d'une analyse fréquentielle sur les données de séparation pour les données d'analyse fréquentielle d'apprentissage et les données de séparation pour les données d'analyse fréquentielle de test, les données de séparation pour les données d'analyse fréquentielle d'apprentissage et les données de séparation pour les données d'analyse fréquentielle de test ayant été soumises à un processus de standardisation ; et
les données d'analyse fréquentielle d'apprentissage et les données d'analyse fréquentielle de test sont générées par le biais d'une analyse en ondelettes.

8. Programme de traitement d'informations exécutable par un ordinateur (100) pour réaliser un traitement, le traitement consistant à :
générer un modèle entraîné par l'utilisation, en tant qu'ensemble de données d'apprentissage de l'apprentissage automatique, de données d'apprentissage incluant des données d'analyse fréquentielle d'apprentissage générées par la réalisation d'une analyse fréquentielle sur des données de séparation générées par un processus de séparation sur un échantillon de sang d'apprentissage dans lequel un type d'hémoglobine est connu, et le type d'hémoglobine connu ;
recevoir des données d'analyse fréquentielle de test générées par la réalisation d'une analyse fréquentielle sur des données de séparation générées par un processus de séparation sur un échantillon de sang de test dans lequel le type d'hémoglobine est inconnu ; et
fournir en sortie le type d'hémoglobine contenu dans l'échantillon de sang de test sur la base du modèle entraîné,
dans lequel :
les données de séparation pour les données d'analyse fréquentielle d'apprentissage et les données de séparation pour les données d'analyse fréquentielle de test sont des chromatogrammes ;
les données d'analyse fréquentielle d'apprentissage et les données d'analyse fréquentielle de test sont générées par la réalisation d'une analyse fréquentielle sur les données de séparation pour les données d'analyse fréquentielle d'apprentissage et les données de séparation pour les données d'analyse fréquentielle de test, les données de séparation pour les données d'analyse fréquentielle d'apprentissage et les données de séparation pour les données d'analyse fréquentielle de test ayant été soumises à un processus de standardisation ; et
les données d'analyse fréquentielle d'apprentissage et les données d'analyse fréquentielle de test sont générées par le biais d'une analyse en ondelettes.
